Europäisches Patentamt

European Patent Office

Office européen des brevets

(19)

(11) **EP 0 948 313 B1**

(12) **FASCICULE DE BREVET EUROPEEN**

(45) Date de publication et mention
de la délivrance du brevet:
**17.04.2002   Bulletin 2002/16**

(51) Int Cl.⁷: **A61K 7/48**, A61K 7/06,
A61K 35/78

(21) Numéro de dépôt: **97951332.2**

(22) Date de dépôt: **15.12.1997**

(86) Numéro de dépôt international:
**PCT/FR97/02296**

(87) Numéro de publication internationale:
**WO 98/26750 (25.06.1998 Gazette 1998/25)**

(54) **UTILISATION D'UN EXTRAIT DE RESINE D'OKOUME, DANS LES DOMAINES COSMETIQUE ET PHARMACEUTIQUE, NOTAMMENT DERMATOLOGIQUES**

**VERWENDUNG EINES AUCOUMEA-HARZ EXTRAKTES ALS KOSMETIKUM UND PHARMAZEUTIKUM, INSBESONDERE ALS DERMATOLOGIKUM**

**USE OF AN EXTRACT OF GABOON RESIN IN COSMETICS AND PHARMACEUTICS, IN PARTICULAR FOR DERMATOLOGICAL PURPOSES**

(84) Etats contractants désignés:
**DE ES FR GB IT**

(30) Priorité:  **16.12.1996  FR 9615448**

(43) Date de publication de la demande:
**13.10.1999   Bulletin 1999/41**

(73) Titulaire: **PARFUMS CHRISTIAN DIOR**
**75008 Paris (FR)**

(72) Inventeurs:
• **RENIMEL, Isabelle**
**F-45470 Trainou (FR)**

• **ANDRE, Patrice**
**F-45170 Neuville aux Bois (FR)**

(74) Mandataire: **Giraud, Françoise**
**Cabinet Beau de Loménie**
**158, rue de l'Université**
**75340 Paris Cedex 07 (FR)**

(56) Documents cités:
• **STN, Serveur de bases de données,
XP002033673 & DELAVEAU ET AL.: PLANTA
MED., vol. 40, 1980, pages 49-54, cité dans la
demande**
• **STN, Serveur de Bases de Données,
XP002033674**

**Description**

L'invention concerne de nouvelles utilisations d'un extrait de résine d'okoumé, dans les domaines cosmétique et pharmaceutique, notamment dermatologique.

Elle concerne plus précisément des utilisations comme agent cosmétique d'un extrait de résine d'okoumé, plus particulièrement pour le soin de la peau, des ongles et des cheveux.

Elle concerne également certaines utilisations dans le domaine de la pharmacie.

L'okoumé est le nom vernaculaire correspondant à la plante Aucoumea klaineana qui est l'unique espèce du genre Aucoumea de la famille des Burseraceae.

L'okoumé est un grand arbre des forêts équatoriales humides de l'Afrique occidentale et centrale. Il est utilisé pour son bois et fournit la majeure partie des bois exportés du Gabon.

Les jeunes rameaux sont couverts d'un duvet ferrugineux. Les feuilles alternes, imparipennées, à folioles ovales allongées, sont glabres et luisantes. Les jeunes feuilles sont de couleur rouge vif ; elles apparaissent à partir du mois d'octobre, jusqu'en décembre-janvier, donnant à la cime une teinte rougeâtre, qui permet en avion de reconnaître les peuplements d'okoumés.

Les inflorescences sont en panicules longs de 10 à 20 cm, à fleurs blanchâtres, presque inodores. La floraison a lieu en octobre-novembre.

Les fruits capsulaires sont glabres ; ils s'ouvrent en 5 lobes coriaces. Les graines sont triangulaires, prolongées par une aile en forme de lame. Les fruits apparaissent vers le mois de février.

La couleur de l'écorce varie du rouge lie de vin au rose saumon en fonction de l'âge de la plante. Cette écorce est légèrement fibreuse, presque granuleuse et, par incision elle laisse exsuder une résine, ou oléorésine, à forte odeur de térébenthine qui devient opaque en se coagulant.

Cette résine contient essentiellement des monoterpènes et des triterpènes à squelette oléanane et tirucallane.

On connaît des utilisations traditionnelles de cette résine : à l'origine la résine d'okoumé était utilisée au Gabon pour fabriquer des flambeaux utilisés lors des cérémonies d'initiation. Cette utilisation, encore persistante de nos jours dans les villages, se prolonge en ville lors de fêtes de famille. Dans les missions, on l'emploie comme succédané de l'encens. En médecine indigène, elle est utilisée pour faire mûrir les abcès et dans le traitement des plaies où elle activerait la cicatrisation.

Sur le plan pharmacologique, l'oléorésine présente un pouvoir anti-bactérien dû, en particulier, à des phénols contenus dans l'huile essentielle.

Pour plus de détails sur les utilisations et les propriétés de l'okoumé ou de son oléorésine, on pourra se reporter aux publications suivantes :

- A.M. Tessier, P. Delaveau, N. Piffault, J. Hoffelt. Planta med. 46, 41 (1982),
- P. Delaveau, P. Lallouette, A.M. Tessier. Planta Med., 40, 49 (1980),
- P. Delaveau, A.M. Vidal-Tessier. Bull. Soc. Bot. Fr., 135, Actualités bot., 3, 25(1988).

Les essais systématiques réalisés par les inventeurs ont permis de mettre en évidence un certain nombre d'actions enzymatiques surprenantes de cette résine, en particulier des actions d'inhibition de plusieurs enzymes, en particulier la kératinase, l'élastase, la phospholipase $A_2$ et la lipoxygénase, ce qui a permis d'envisager son utilisation dans des produits cosmétiques, notamment des produits destinés au soin de la peau, des ongles et des cheveux.

La kératinase est une enzyme produite sur le corps essentiellement par des champignons. Elle est produite industriellement à partir de Streptomyces fradiae. Son action sur la peau et les phanères conduit à une dégradation des kératines, ce qui occasionne notamment une désorganisation de la structure des phanères (ongles blancs et striés, cheveux cassants, etc.).

L'élastase, enzyme de dégradation de l'élastine, est présente dans les cellules, notamment dans les cellules dermiques (fibroblastes) ainsi que, dans une moindre mesure, dans les cellules de l'épiderme (kératinocytes). On a observé que la quantité et l'activité de l'élastase augmentent au cours du processus de vieillissement cutané, aussi bien intrinsèque qu'actinique. Par une dégradation des fibres d'élastine, l'action de l'élastase a pour conséquence une perte de l'élasticité cutanée, un relâchement de la peau et l'apparition de rides.

La phospholipase $A_2$ ($PLA_2$) est une enzyme produite par les cellules au niveau membranaire. Elle prédomine dans les cellules liées aux phénomènes d'inflammation, telles que les mastocytes. Par son action, elle libère l'acide arachidonique lié aux phospholipides membranaires. Cet acide se métabolise ensuite en différents médiateurs lipidiques de l'inflammation et de l'allergie, tels que les leucotriènes et les prostaglandines.

La 5'-lipoxygénase, désignée ci-après par "lipoxygénase", est, comme la $PLA_2$, une enzyme membranaire. Elle intervient dans la "cascade de l'inflammation" en aval de la libération de l'acide arachidonique par la $PLA_2$, dans la conversion de cet acide en leucotriènes, médiateurs de l'inflammation.

Ainsi, il a été mis en évidence par les inventeurs, que, grâce à leur action inhibitrice sur les enzymes précitées, les extraits de résine d'okoumé présentaient un grand intérêt en cosmétique et en thérapeutique.

Plus particulièrement, l'invention fournit des compositions destinées au soin des ongles, des cheveux et des cils, à la lutte contre les effets du vieillissement sur la peau, à la prévention ou au traitement des inflammations et des allergies cutanées.

En effet, par la découverte de l'activité des extraits de résine d'okoumé inhibant l'action d'enzymes, l'invention fournit différentes solutions dans le domaine de la cosmétique et de la thérapeutique, notamment de la dermatologie. S'agissant de l'inhibition de la kératinase, les compositions selon l'invention permettent de prévenir et de traiter la détérioration des structures kératiniques, notamment celles des phanères, en particulier des ongles, des cheveux et des cils. S'agissant de l'inhibition de l'élastase, les compositions selon l'invention s'opposent à la dégradation des fibres d'élastine et, de ce fait, ces compositions maintiennent les qualités biomécaniques de la peau, en particulier ses qualités d'élasticité au niveau du derme, et permettent de lutter contre le relâchement de la peau et l'apparition des rides. S'agissant de l'inhibition de la phospholipase A$_2$ d'une part et de la lipoxygénase d'autre part, les compositions selon l'invention agissent ainsi doublement dans le processus de formation des médiateurs de l'allergie cutanée et de l'inflammation, en limitant ou en bloquant ce processus.

Selon un autre avantage de l'invention, la résine d'okoumé s'est avérée soluble dans de nombreux solvants organiques. Elle est même soluble presque en totalité dans des esters tels que l'acétate d'éthyle et l'acétate de butyle, ce qui rend son intérêt encore plus grand pour la fabrication de vernis à ongles, qui utilisent très généralement ces solvants, en particulier pour la fabrication de vernis à ongles traitants.

Un autre avantage de l'utilisation d'extraits de résine d'okoumé dans les produits de traitement pour les ongles, notamment dans les vernis à ongles, tient à ce que ces extraits possèdent en eux-même un caractère filmogène lorsqu'ils sont appliqués sur une surface, notamment celle de l'ongle.

Par ailleurs, les qualités filmogènes de la résine d'okoumé peuvent également être mises à profit dans des compositions destinées à une application sur les cheveux ou sur les cils, en particulier dans des compositions destinées à la coiffure telles que laques, gels ou émulsions coiffants, ou encore dans des compositions de mascaras pour améliorer le gainage des cils.

D'autres avantages de l'invention apparaissent au vu de la description et des exemples qui suivent.

Ainsi, selon l'une de ses caractéristiques essentielles, l'invention concerne des compositions cosmétiques contenant un l'extrait de résine d'okoumé en présence d'un véhicule cosmétiquement acceptable.

L'extrait est avantageusement obtenu par macération de la résine dans un solvant de polarité moyenne ou faible, suivie d'une filtration du mélange. Le solvant de la solution obtenue peut être au besoin évaporé pour obtenir l'extrait sec.

Par solvant de polarité moyenne ou faible, on entend un solvant présentant un paramètre de polarité inférieur ou égal à 6, un tel indice étant défini dans Pratical High-Performance Liquid Chromatography, V.R. Meyer, 1988, p. 120-121.

De préférence, l'évaporation sera réalisée sous pression réduite.

A titre de solvants avantageusement utilisés, on citera :

- les hydrocarbures aliphatiques en C$_6$ à C$_{12}$, tels que l'hexane et l'heptane
- des solvants chlorés, notamment le dichlorométhane
- les éthers,

tels que l'éther éthylique ou diisopropylique

- l'acétone
- les esters tels que l'acétate d'éthyle et l'acétate de butyle
- des alcools en C$_1$ à C$_4$, tels que le méthanol, l'éthanol et l'isopropanol.

On pourra également obtenir l'extrait de résine d'okoumé par la technique dite de l'extraction au dioxyde de carbone supercritique.

Le rendement de l'extraction dépend essentiellement de l'âge de l'arbre.

Ainsi, en utilisant comme solvant l'acétate d'éthyle, on obtiendra un rendement d'extraction compris entre 90 et 98% selon l'âge de l'arbre.

Selon un mode de réalisation avantageux, cette composition comprend de 0,01 à 10 % en poids et en particulier de 0,2 à 1 % en poids d'extrait sec de résine d'okoumé par rapport au poids total de la composition finale.

Selon d'autres modes de réalisation avantageux de l'invention, la composition est formulée pour une application topique sur la peau, sur les cheveux, les cils ou sur les ongles.

Par ailleurs, les essais réalisés par les inventeurs ont clairement montré que, non seulement le rendement d'extraction était lié à la nature du solvant utilisé mais également l'activité enzymatique de l'extrait. Les exemples joints en annexe montrent clairement l'effet du choix du solvant sur l'activité enzymatique de l'extrait.

La quantité d'extrait sera également choisie en fonction de l'effet filmogène souhaité. En effet, selon un avantage particulier lié à la nature du produit, l'extrait permet de conférer à la composition des qualités filmogènes ou de les améliorer.

Les compositions selon l'invention peuvent être formulées selon toute forme acceptable pour leur emploi en cosmétologie. Il peut, en particulier, s'agir d'une crème, en particulier d'une crème pour le visage ou pour les mains, d'un gel en particulier un gel coiffant, d'un baume, notamment un baume démêlant, d'un mascara, d'un fond de teint ou d'une préparation pour les ongles, telles qu'une base traitante ou un vernis à ongles traitant.

Selon un autre aspect, l'invention concerne l'utilisation de la résine d'okoumé comme agent cosmétique, ledit agent étant incorporé dans une composition cosmétique telle que définie précédemment.

La quantité de cet agent pourra être adaptée en fonction en particulier des qualités filmogènes recherchées pour la composition.

Les compositions cosmétiques de l'invention seront utilisées dans tout domaine lié au soin ou au maquillage.

Cet agent cosmétique sera notamment utilisé dans toutes les applications où l'on recherche, en particulier, à inhiber l'action de la kératinase et/ou de l'élastase et/ou de la phospholipase $A_2$ et/ou de la lipoxygénase.

De telles compositions seront, en particulier, destinées au soin des ongles, des cheveux ou des cils, en particulier pour les maintenir en bon état quant à leur structure kératinique, pour faciliter la coiffure ou pour améliorer le maquillage des cils.

Elles seront également utilisées pour lutter contre les effets du vieillissement sur la peau, notamment en préservant ou améliorant les qualités biomécaniques de la peau, en particulier son élasticité, en retardant l'apparition des rides ou en diminuant leur profondeur et en améliorant la fermeté cutanée.

Elles seront encore utilisées pour lutter contre les effets nocifs des radicaux libres sur la peau, pour le soin ou le maquillage des peaux sensibles, notamment en atténuant ou en supprimant les phénomènes d'irritation, d'inflammations ou d'allergies qui se traduisent en général au niveau cutané par des rougeurs, des sensations de brûlure ou de picotements.

Ainsi, les compositions cosmétiques de l'invention seront notamment utilisées pour toutes les applications cosmétiques où l'on cherche à inhiber l'activité des enzymes précitées.

C'est le cas, en particulier des compositions destinées au soin des cheveux, des cils ou des ongles où l'on recherche tout particulièrement cette activité anti-kératinase.

Ainsi, selon un autre aspect, l'invention concerne des compositions cosmétiques destinées au soin de la peau, en particulier pour lutter contre le vieillissement cutané ou l'inflammation.

Selon un autre aspect encore de l'invention, elle concerne des compositions cosmétiques destinées au soin des cheveux. Il pourra, en particulier, s'agir de laques, de gels ou d'émulsions coiffants.

L'invention concerne également des produits de maquillage, en particulier de maquillage traitant, tels que les vernis à ongles, les mascaras ou les fonds de teint.

Selon un aspect tout particulièrement intéressant de l'invention, elle concerne également des compositions cosmétiques destinées au traitement des ongles. En effet, la kératine, protéine de structure, est prédominante dans l'ongle et lui confère sa rigidité. Lors du vieillissement, les kératines deviennent moins rigides, ce qui se traduit par des ongles plus mous, plus friables et aussi plus vulnérables à l'attaque de certaines protéases comme la kératinase. Les compositions de l'invention peuvent donc être utilisées avantageusement pour le soin des ongles, du fait en particulier de leur action d'inhibition de la kératinase démontrée par les essais effectués par les inventeurs.

Outre ses propriétés d'inhibition de la kératinase et sa solubilité quasi-totale dans des solvants utilisés dans la préparation des vernis à ongles, la résine d'okoumé présente l'avantage d'améliorer le caractère filmogène de ces compositions, contribuant grandement ainsi aux propriétés rhéologiques et aux qualités de ces vernis après leur application sur les ongles.

Comme on l'a vu précédemment, l'efficacité des compositions cosmétiques précédemment décrites a pu être corrélée avec un certain nombre d'activités enzymatiques. La mise en évidence de ces activités enzymatiques a permis d'envisager également l'utilisation des extraits définis précédemment pour la préparation de compositions pharmaceutiques, notamment dermatologiques dans lesquelles de telles activités sont souhaitées. Les essais réalisés par les inventeurs de la présente invention ont confirmé l'efficacité de ces compositions pharmaceutiques.

Ainsi, l'inhibition de la phospholipase $A_2$ et l'inhibition de la 5'-lipoxygénase ont pu être corrélées avec une efficacité dans la prévention et le traitement des phénomènes d'inflammation.

Par ailleurs, l'activité d'inhibition de la kératinase a pu être corrélée également à une activité dans le domaine du traitement des désordres affectant la kératine, notamment au niveau des ongles, des cils et des cheveux.

Ainsi, selon une autre caractéristique essentielle, l'invention concerne également l'utilisation d'un extrait de résine d'okoumé pour la préparation d'une composition pharmaceutique, notamment dermatologique, destinée à la prévention ou au traitement des affections des phanères, en particulier des ongles, se traduisant par une dégradation des structures kératiniques, telles que les onychomycoses, au traitement des effets du vieillissement intrinsèque ou actinique de la peau, au traitement des effets nocifs des radicaux libres sur la peau, à la prévention ou au traitement des manifestations cutanées des allergies et des inflammations, ledit extrait étant incorporé dans un véhicule pharmaceutiquement acceptable.

L'invention concerne également des procédés de traitement thérapeutique, notamment dermatologique, destinés à la prévention ou au traitement des affections des phanères, en particulier des ongles, se traduisant par une dégradation des structures kératiniques, telles que les onychomycoses, au traitement des effets du vieillissement intrinsèque ou actinique de la peau, au traitement des effets nocifs des radicaux libres sur la peau, à la prévention ou au traitement des manifestations cutanées des allergies et des inflammations, selon lesquels on applique sur la partie du corps à traiter une quantité efficace d'une composition pharmaceutique contenant un extrait de résine d'Okoumé.

Pour ces différentes applications, la composition pharmaceutique présente avantageusement une activité inhibitrice de la kératinase et/ou de l'élastase et/ou de la phospholipase $A_2$ et/ou de la lipoxygénase.

Selon une variante, l'invention concerne l'utilisation d'un extrait de résine d'okoumé pour la préparation d'une composition pharmaceutique, notamment dermatologique destinée au traitement et à la prévention des manifestations allergiques, en particulier de l'allergie cutanée ou au traitement des effets nocifs des radicaux libres.

Ces deux types d'application sont directement liés à l'inhibition des médiateurs lipidiques de l'inflammation.

Dans ces applications du domaine pharmaceutique, notamment dermatologique, les compositions utilisées sont de préférence des compositions à application topique destinées à être appliquées en particulier sur la peau et les phanères. Par ailleurs, les extraits de résine d'okoumé utilisés pour leur préparation sont obtenus de la même façon que les extraits utilisés dans le domaine cosmétique et sont introduits dans un véhicule pharmaceutiquement, notamment dermatologiquement, acceptable à des concentrations comprises entre 0,01 % et 10 % en poids, et en particulier entre 0,2 % et 1 % en poids, d'extrait sec de ladite résine.

Comme dans le cas des applications cosmétiques, on choisira le solvant d'extraction en fonction du type d'activité enzymatique que l'on souhaitera privilégier dans l'action de la composition pharmaceutique.

Les compositions pharmaceutiques, notamment dermatologiques, de l'invention pourront, en outre, contenir différentes substances actives pharmacologiquement utilisables dans le traitement des pathologies des phanères, en particulier des ongles.

Elles pourront, en particulier, contenir des agents connus pour leur activité anti-fongique, notamment des agents actifs sur les dermatophytes responsables, en particulier, des onychomycoses.

Parmi ces agents, on peut citer le sel d'éthanolamine du ciclopirox, également dénommé ciclopirox olamine.

Les exemples qui suivent sont donnés à titre purement illustratif de l'invention. En référence à ces exemples, sont données également les figures 1 à 4 qui représentent respectivement :

- figure 1 : l'évolution de l'absorbance à 280 nm en fonction du temps en présence d'un milieu réactionnel contenant de la kératinase, des morceaux d'ongles et en présence (courbe 1) ainsi qu'en absence (courbe 2) de résine d'okoumé. Cette figure est donnée en référence à l'exemple 3 ;
- figure 2 : une photographie en microscopie électronique à grossissement x 350 d'un ongle non traité ;
- figure 3 : une photographie en microscopie électronique à grossissement x 350 d'un ongle après traitement avec de la kératinase pendant 48 h ;
- figure 4 : une photographie en microscopie électronique à grossissement x 350 d'un ongle après le même traitement que dans le cas de la figure 3, mais en présence également d'extrait de résine d'okoumé.

Sauf indication contraire, les proportions données dans les exemples de compositions sont exprimées en pourcentage en poids

Exemple 1

Préparation d'un extrait d'oléorésine d'okoumé selon l'invention

100 g d'oléorésine préalablement broyée sont introduits dans 1 000 ml d'acétate d'éthyle. La suspension est laissée, à température ambiante sous agitation modérée, pendant 3 heures. On filtre ensuite le mélange, puis on évapore le solvant du filtrat ainsi obtenu sous pression réduite. On récupère alors environ 95 g d'extrait sec de résine d'okoumé présentant l'aspect d'un résidu sirupeux. Pour préparer les compositions selon l'invention, il est possible d'utiliser soit la solution d'extrait de résine, éventuellement concentrée, dans le solvant d'extraction, soit l'extrait sec. Ainsi, pour la préparation de vernis à ongles selon l'invention, il sera possible d'utiliser directement une solution de la résine dans l'acétate d'éthyle ou de butyle. Par exemple, on pourra utiliser une solution à 10 % en extrait de résine.

Exemple 2

Mise en évidence de l'activité inhibitrice d'oléorésine d'okoumé sur différentes enzymes

2.1 Extrait utilisés

Les tests d'inhibition enzymatiques étant menés en milieu aqueux, il est nécessaire d'utiliser des solvants d'extraction miscibles avec l'eau.

Ainsi, en procédant comme décrit dans l'exemple 1 mais avec d'autres solvants, on a réalisé trois extraits différents avec respectivement de l'eau, du méthanol et du DMSO. La concentration de ces extraits est ensuite ajustée à 0,5 % en extrait sec de résine, soit par ajout soit par évaporation du solvant d'extraction.

Tous les tests décrits ci-après ont été réalisés en trois exemplaires. Les valeurs rapportées sont des moyennes arithmétiques.

2.2. Inhibition de l'élastase

a) Principe du test :

Les techniques de mise en évidence de l'inhibition de l'élastase ont été décrites par différents auteurs (BAUMSTARK, J.S. et al., Biochim. Biophys. Acta (1963), 77, 676; BIETH, B. et al., Biochem. Med., (1974), 11, 350; C. FRANCK, I. BYRJALSEN, Biol. Chem. Hoppe Seyler, (1988) 369 (8) 677-82).

Le principe actif est le suivant : un substrat est mis en présence d'élastase en milieu aqueux, puis, après incubation, on effectue une mesure des produits de la réaction.

En l'occurrence, le substrat est le N-succinyl-(Ala)$_3$-para-nitroanilide, disponible auprès de la Société Sigma (Réf. : S 4760) en solution à 0,5 mg/ml dans un tampon Tris-HCl 0,2 M à pH 8,8. L'élastase ajoutée au milieu réactionnel libère la para-nitroaniline et le résidu peptidique. L'évolution de la réaction est observée au spectrophotomètre Uvikon 941® (Kontron S.A.) à la longueur d'onde $\lambda$ de 379 nm.

La composition du milieu réactionnel est la suivante :

- solution de substrat (0,5 mg/ml de tampon) : 200 $\mu$l
- tampon Tris-HCl        : 600 $\mu$l
- solvant d'extraction        : 100 $\mu$l

Le solvant d'extraction contient ou non l'effecteur, c'est-à-dire l'extrait de résine d'okoumé, à la concentration de 0,5 % en poids, selon qu'il s'agit de l'essai avec effecteur, ou de l'essai sans effecteur (activité basale de l'enzyme).

A ces 900 $\mu$l de milieu réactionnel, on ajoute 100 $\mu$l de la solution d'enzyme à raison de 35 U/ml dans le tampon Tris-HCl.

On mesure alors la cinétique de la libération de para-nitroaniline par l'absorption d'une lumière mono-chromatique de longueur d'onde 379 nm au moyen du spectrophotomètre, ce qui permet de calculer le pourcentage d'inhibition $I_E$ suivant la formule -ci-dessous :

$$I_E = \frac{\Delta AbB / mn - \Delta AbE / mn}{\Delta AbB / mn} \times 100$$

dans laquelle $\Delta AbB / mn$ est la différence d'absorbance par minute du milieu réactionnel pour l'activité basale, et $\Delta AbE / mn$, la différence d'absorbance du milieu réactionnel pour l'essai avec effecteur. Les résultats figurent au tableau I ci-après.

2.3. Inhibition de la phospholipase A$_2$

Selon le principe du test (UTHE, J.F. and MAGEE, W.L., Can. J. Biochem. (1971), 49, 776 ; DENNIS, E.A., J. Lipids Res., (1973), 14, 152), l'enzyme est mise en présence d'un phospholipide. Celui-ci est alors transformé en lysolécithine avec libération d'un acide gras, insoluble dans le milieu réactionnel. Cette réaction peut donc être suivie par turbidimétrie au moyen d'un spectrophotomètre, en utilisant par exemple la longueur d'onde de 360 nm.

La composition du milieu réactionnel est indiquée ci-après. Toutes les solutions sont réalisées dans de l'eau distillée.

- solution à 14 mM de L-phosphatidylcholine dimyristoyl (substrat) : 600 $\mu$l

- solution de chlorure de sodium 0,67 M : 150 µl
- solution de chlorure de calcium 66 mM : 200 µl
- eau distillée : 850 µl
- solvant, avec ou sans effecteur à 0,5 % : 100 µl

A ce milieu, on ajoute 100 µl de solution d'enzyme dans l'eau distillée à la concentration de 96 U/ml.

Comme dans le cas de l'inhibition de l'élastase, la cinétique réactionnelle est suivie au moyen d'un spectrophotomètre Uvikon 941® , à la longueur d'onde de 360 nm.

On détermine le pourcentage d'inhibition $I_p$ de la phospholipase $A_2$ par calcul suivant une formule analogue à celle de l'inhibition de l'élastase

$$I_p = \frac{\Delta AbB / mn - \Delta AbE / mn}{\Delta AbB / mn} \times 100$$

dans laquelle $\Delta AbB$ et $\Delta AbE$ ont la même signification que précédemment.

Les résultats figurent au tableau I ci-après.

2.4. Inhibition de la 5'-lipoxygénase

Comme cela a été exposé plus haut, la lipoxygénase intervient dans la formation de médiateurs de l'inflammation, en particulier les leucotriènes, à partir de l'acide arachidonique.

Dans l'essai de mise en évidence de l'inhibition de cette enzyme (C.W. Taylor, H.R. Morris, Brit. Med. Bull. (1989) 39 219-222 ; J. Magee, Methods of Enzymatic Analysis, H.U. Bergmayer Ed., (1965) p. 411-4, Academic Press, N.Y.), on utilise de l'acide linoléique comme substrat qui se transforme en présence de l'enzyme en acide 5-hydroperoxy-6,8,11,14-éicosatétraénoïque (5 HPETE).

Composition du milieu réactionnel :

- acide linoléique (substrat), solution 0,5 mM : 2 000 µl
- tampon borate 0,2 M à pH 9 : 950 µl
- solvant, avec ou sans effecteur à 0,5 % : 100 µl

dans le tampon borate

Pour préparer la solution d'acide linoléique ci-dessus, on réalise préalablement une salification partielle de l'acide linoléique dans du méthanol en présence de soude, puis on l'introduit dans le tampon borate.

On ajoute ensuite 100 µl de solution de l'enzyme 5'-lipoxygénase dans du tampon borate à la concentration de 10 U/ml,

Comme dans les essais précédents, on mesure l'absorbance des milieux en fonction du temps, et on calcule le pourcentage d'inhibition $I_L$ de la 5'-lipoxygénase.

Les résultats figurent au tableau I ci-dessous.

TABLEAU I

| Taux d'inhibition d'enzymes | | | |
|---|---|---|---|
| | élastase $I_E$ | phospholipase $A_2$ $I_p$ | 5'-lipoxygénase $I_L$ |
| $E_E$ | 17% | 25% | 18% |
| $E_M$ | 72% | 22% | 32% |
| $E_D$ | 75% | 43% | 61% |

$E_E$ : extrait aqueux d'oléorésine d'okoumé, à 0,5 % dans l'eau (solvant)

$E_M$ : extrait méthanolique d'oléorésine d'okoumé, à 0,5 % dans le méthanol (solvant)

$E_D$ : extrait au DMSO d'oléorésine d'okoumé, à 0,5 % dans le DMSO (solvant)

Les résultats obtenus pour l'inhibition peuvent être considérés comme significatifs pour des valeurs de l'inhibition supérieures à 25 %.

La fraction de résine d'okoumé extraite par le DMSO présente une activité inhibitrice très significative sur les trois enzymes. La fraction méthanolique présente une activité inhibitrice équivalente de celle de la fraction DMSO pour l'élastase, ainsi qu'une activité inhibitrice notable pour la 5'-lipoxygénase. Par contre, cette fraction n'est que très faiblement active sur l'inhibition de la PLA$_2$. Quant à la fraction aqueuse, elle n'est que très faiblement inhibitrice pour les trois enzymes testées.

On doit conclure de ces résultats expérimentaux que les principes actifs présents dans la résine d'okoumé et extraits par les solvants organiques testés ne sont pas extraits par des solvants trop polaires tels que l'eau.

Ces résultats démontrent l'intérêt des extraits de résine d'okoumé selon l'invention, dans les domaines de la cosmétique et de la pharmacie, notamment de la dermatologie, dès qu'il s'agit de lutter contre les effets négatifs dus à l'action de ces enzymes.

## Exemple 3

### Protection de la kératine unguéale

500 mg de morceaux d'ongles préalablement coupés sont mis en présence de kératinase avec ou sans extrait de résine d'okoumé pendant 3 jours sous agitation à 37°C. Des prélèvements pour le dosage des protéines sont effectués à 6, 12, 24, 48 et 72 heures et les protéines relarguées sont dosées par mesure d'absorbance. Dans ces essais, l'extrait de résine d'okoumé est réalisé conformément à l'exemple 1, et est utilisé en solution à 1 % dans l'acétate d'éthyle.

Le tableau 2 ci-dessous précise la composition des milieux réactionnels pour les essais réalisés.

La solution tampon utilisée est une solution tampon Tris-HCl 0,1 M, pH 7,5. La concentration des solutions d'enzyme dans le tampon est de 480 U/ml. L'enzyme est désactivée par chauffage à 100°C pendant 10 mn.

On notera qu'un essai "blanc enzyme", c'est-à-dire avec l'enzyme désactivée, est nécessaire pour pouvoir prendre en compte l'absorbance à 280 nm liée au seul apport d'enzyme.

## TABLEAU 2

| | Blanc enzyme | Activité basale | Traitement okoumé |
|---|---|---|---|
| Ongle (mg) | 500 | 500 | 500 |
| Enzyme désactivée | 100 µl | - | - |
| Enzyme active | - | 100 µl | 100 µl |
| Acétate d'éthyle | 200 µl | 200 µl | - |
| Solution d'extrait d'okoumé | - | - | 200 µl |
| Tampon | 1 ml | 1 ml | 1 ml |

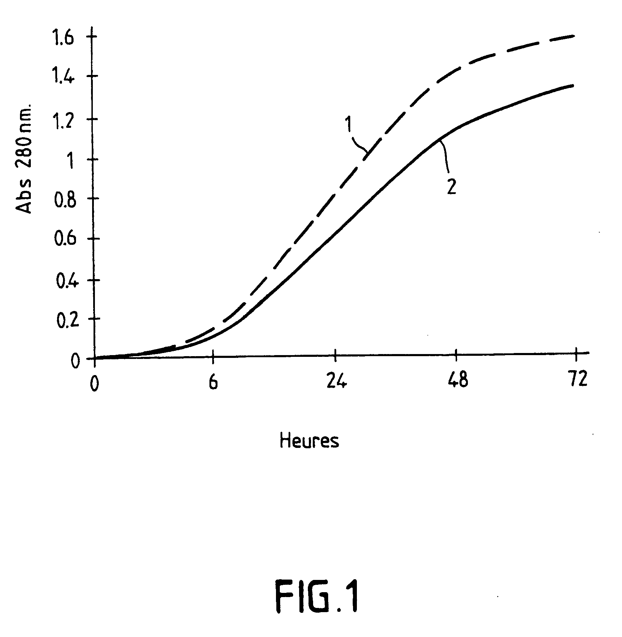

La quantité de protéines relarguées dans le milieu réactionnel est proportionnelle à l'absorbance mesurée à 280 nm au moyen d'un spectrophotomètre Uvikon 941® . Les résultats des mesures d'absorbance sont donnés sur la figure 1 dans laquelle la courbe en pointillé (courbe 1) correspond à l'absorbance en présence de kératinase seule et la courbe en trait plein (courbe 2), l'absorbance en présence de kératinase et d'extrait d'okoumé. A 24 h, l'absorbance du milieu contenant l'extrait d'okoumé est inférieure de 12 % à celle du milieu témoin (activité basale). A 48 h, l'inhibition est de 25 %, pour se stabiliser à environ 28 % à partir de 72 h. Il apparaît clairement de ces résultats que la présence d'extrait de résine d'okoumé inhibe l'action de la kératinase, protégeant ainsi la kératine unguéale.

## Exemple 4

### Mise en évidence de l'effet filmogène

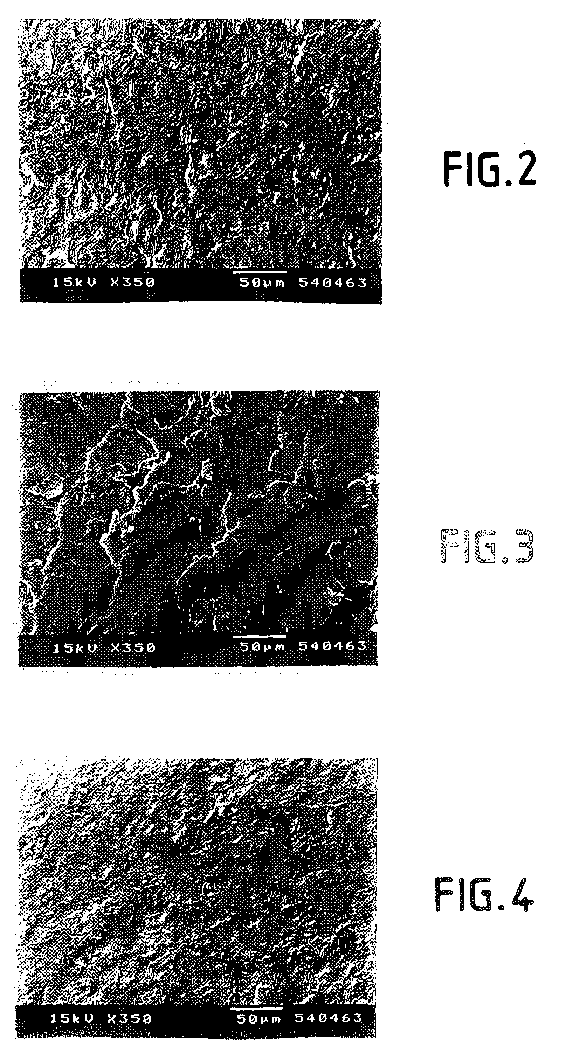

Des observations faites par microscopie électronique sur les morceaux d'ongles après 48 h de mise en contact avec les milieux réactionnels de l'essai d'inhibition de la kératinase décrit à l'exemple précédent, permettent de visualiser la différence entre un ongle témoin, un ongle exposé à une kératinase avec ou sans traitement de protection par l'okoumé. Ces résultats apparaissent clairement sur les photographies réalisées par microscopie électronique d'une part d'un ongle témoin (figure 2), d'autre part d'un ongle traité par la kératinase (figure 3) et d'autre part d'un ongle traité par la kératinase en présence de résine d'okoumé (figure 4). Sur ces photographies, il apparaît clairement que, non seulement la présence de résine d'okoumé protège l'ongle de l'attaque de la kératinase mais également amène un effet filmogène qui se traduit par un aspect plus lisse de l'ongle.

Exemple 5

Pommade dermatologique pour les ongles

- Extrait sec de résine d'okoumé selon l'exemple 1      0,5 g
- Excipient gras (alcools gras polyoxyéthylénés, glycérol, huile de maïs inter-estérifié, lauryl trihydroxy-3,4,5-benzoate, sorbitol et polyglycol stéarate mixte, eau purifiée)      qsp 100 g

Cette pommade peut être appliquée matin et soir sur les ongles abîmés, en particulier comme traitement d'accompagnement anti-mycosique.

Exemple 6

Crème dermatologique pour les ongles

- Extrait sec de résine d'okoumé selon l'exemple 1      1,0 g
- Sel d'éthanolamine du ciclopirox      1,0 g
- Excipient émulsifié huile-dans-eau      qsp 100 g

Cette crème associant un extrait de résine d'okoumé et un agent anti-fongique présente un grand intérêt dans le traitement des onychomycoses à dermatophytes.

Exemple 7

Gel cosmétique anti-rides

- Extrait sec de résine d'okoumé, fraction méthanolique selon l'exemple 2      0,05 g
- Carbomer 0,3 g
- Glycérine 3,0 g
- EDTA tétrasodique      0,05 g
- Eau florale d'Hammamelis      3,00 g
- Polyméthylméthacrylate      1,00 g
- Parfums, conservateurs, colorant, neutralisant      qs
- Eau distillée      qsp100 g

Ce gel présente un effet anti-rides et apaisant.

Exemple 8

Baume démêlant, à rincer

- Extrait sec de résine d'okoumé selon l'exemple 1      1,0 g
- Alcool cétostéarylique + cétéaryl glucoside      5,0 g
- Quatemium - 82      2,0 g
- Cocamidopropyl bétaïne      3,0 g
- Neutralisant      qsp pH 5,5

- Parfums, conservateur, colorant      qs
- Eau      qsp100 g

Exemple 9

Crème anti-rides pour le visage

- Extrait sec de résine d'okoumé selon l'exemple 1      0,5 g
- Glycéryl stéarate + PEG 100 stéarate      5,0 g
- Alcool cétylique      1,0
- Alcool stéarylique      1,0
- Cire d'abeille      1,50
- Squalane      3,0
- Polyisobutène hydrogéné      4,0
- Octanoate de cétéaryle      1,50
- Tricaprylate/caprate de glycérol      3,0
- Diméthicone      1,0
- Gomme xanthane      0,2
- Carbomer 0,15
- Glycérine 2,0
- Neutralisant, conservateur, parfums, colorants      qs
- Eau      qsp 100

Exemple 10

Crème pour peau sensible

- Extrait sec de résine d'okoumé, fraction méthanolique selon l'exemple 2      0,2 g
- Méthylglucose sesquistéarate      3,0
- Cire d'abeille      3,0
- Alcool béhénique      3,0
- Octanoate d'octyle      5,0
- Huile minérale fluide      7,5
- Octanoate de cétostéaryle      5,0
- Glycérine 3,0
- Gomme xanthane      0,50
- Parfums      0,30
- Conservateur, colorants      qs
- Eau      qsp100

Exemple 11

Base traitantee pour les ongles

- Extrait sec de résine d'okoumé à 10 % dans l'acétate d'éthyle, selon l'exemple 1      10
- Acétate d'éthyle      20,5
- Acétate de butyle      30,5
- Résine arylsulfonamide      9,1
- Camphre      1,9
- Nitrocellulose à 70 % dans l'isopropanol      18,5
- Dibutylphtalate      5,5
- Stéaralkonium hectorite      1,3

- Solution acide citrique à 10 % dans l'isopropanol    0,6
- Isopropanol    2,1

Exemple 12

Vernis à ongles traitant

- Extrait sec de résine d'okoumé à 10 % dans l'acétate d'éthyle, selon l'exemple 1    20
- Acétate d'éthyle    10
- Acétate de butyle    30
- Résine polyester    9,1
- Camphre    1,9
- Nitrocellulose à 70 % dans l'isopropanol    18,5
- Dibutylphtalate    5,5
- Stéaralkonium hectorite    1,3
- Solution acide citrique à 10 % dans l'isopropanol    0,6
- Isopropanol    2,1
- pigments    1


**Revendications**

1. Composition cosmétique, **caractérisée par le fait qu'**elle contient un extrait de résine d'okoumé en présence d'un véhicule cosmétiquement acceptable.

2. Composition selon la revendication 1, **caractérisée en ce que** ledit extrait est obtenu par macération de ladite résine dans un solvant ou un mélange de solvants, dont le paramètre de polarité est inférieur ou égal à 6, suivie d'une filtration et, au besoin, d'une évaporation du solvant.

3. Composition selon la revendication 2, **caractérisée en ce** ledit solvant organique est choisi dans le groupe constitué des hydrocarbures aliphatiques en $C_6$ à $C_{12}$ tels que l'hexane et l'heptane, des solvants chlorés, notamment le dichlorométhane, des éthers tels que l'éther éthylique ou diisopropylique, l'acétone, des esters, tels que l'acétate d'éthyle et l'acétate de butyle, des alcools en $C_1$ à $C_4$, tels que le méthanol, l'éthanol et l'isopropanol.

4. Composition selon la revendication 1, **caractérisée en ce que** ledit extrait est obtenu par extraction au dioxyde de carbone supercritique.

5. Composition selon l'une des revendications précédentes, **caractérisée en ce qu'**elle comprend de 0,01 % à 10 % en poids, et en particulier de 0,2 % à 1 % en poids, d'extrait sec de ladite résine.

6. Composition selon l'une des revendications 1 à 5, **caractérisée en ce qu'**elle comprend une quantité efficace dudit extrait pour conférer des qualités filmogènes à ladite composition ou les améliorer.

7. Composition selon l'une des revendications 1 à 6, **caractérisée en ce qu'**elle se présente sous forme d'une crème, en particulier pour le visage ou pour les mains, d'un gel, en particulier un gel coiffant, d'un baume, notamment un baume démêlant, d'un mascara, d'un fond de teint ou d'une préparation pour les ongles, telle qu'une base traitante ou un vernis à ongles traitant.

8. Utilisation d'un extrait de résine d'okoumé comme agent cosmétique, ledit agent étant incorporé dans une composition cosmétique telle que définie dans l'une des revendications 1 à 7.

9. Utilisation selon la revendication 8, **caractérisée en ce que** ledit agent cosmétique est incorporé dans ladite composition en quantité suffisante pour conférer des qualités filmogènes à ladite composition ou les améliorer.

10. Utilisation selon l'une des revendications 8 ou 9, **caractérisée en ce que** ladite composition est une composition de soin ou de maquillage.

**11.** Utilisation selon l'une des revendications 8 à 10, **caractérisée en ce que** ledit agent cosmétique contenu dans ladite composition présente une action inhibitrice de la kératinase et/ou de l'élastase et/ou de la phospholipase $A_2$ et/ou de la lipoxygénase.

**12.** Utilisation selon l'une des revendications 8 à 11, **caractérisée en ce que** ladite composition est destinée au soin des ongles, des cheveux ou des cils, en particulier pour les maintenir en bon état quant à leur structure kératinique, pour faciliter la coiffure ou pour améliorer le maquillage des cils.

**13.** Utilisation selon l'une des revendications 8 à 12, **caractérisée en ce que** ladite composition est destinée à lutter contre les effets du vieillissement sur la peau, notamment en préservant ou améliorant les qualités biomécaniques de la peau, en particulier son élasticité, en retardant l'apparition des rides ou en diminuant leur profondeur, et en améliorant la fermeté cutanée.

**14.** Utilisation selon l'une des revendications 8 à 13, **caractérisée en ce que** ladite composition est destinée à lutter contre les effets noctifs des radicaux libres sur la peau, ainsi qu'au soin ou au maquillage des peaux sensibles, notamment en atténuant ou en supprimant les phénomènes d'irritation, d'inflammation ou d'allergie, qui se traduisent généralement au niveau cutané par des rougeurs, des sensations de brûlure ou de picotements.

**15.** Utilisation d'un extrait de résine d'okoumé pour la préparation d'une composition pharmaceutique, notamment dermatologique, destinée à la prévention ou au traitement des affections des phanères se traduisant par une dégradation des structures kératiniques, au traitement des effets du vieillissement intrinsèque ou actinique de la peau, au traitement des effets nocifs des radicaux libres sur la peau, à la prévention ou au traitement des manifestations cutanées des allergies et des inflammations, ledit extrait étant incorporé dans un véhicule pharmaceutiquement acceptable.

**16.** Utilisation selon la revendication 15, **caractérisée en ce que** la composition présente une activité inhibitrice de la kératinase et/ou de l'élastase et/ou de la phospholipase $A_2$ et/ou de la lipoxygénase.

**17.** Utilisation selon l'une des revendications 15 ou 16, **caractérisée en ce que** ledit extrait est utilisé pour la préparation d'une composition destinée au traitement ou à la prévention des pathologies des phanères.

**18.** Utilisation selon l'une des revendications 15 à 17, **caractérisée en ce que** ladite composition pharmaceutique contient en outre au moins une substance active connue pour son activité antifongique, notamment un agent actif sur les dermatophytes responsables des onychomycoses.

**19.** Utilisation selon l'une des revendications 15 à 18, **caractérisée en ce que** ledit extrait contenu dans ladite composition est obtenu suivant un procédé tel que défini dans l'une des revendications 2 à 4.

**20.** Utilisation selon l'une des revendications 15 à 19, **caractérisée en ce que** ladite composition contient de 0,01 % à 10 % en poids, et en particulier de 0,2 % à 1 % en poids, d'extrait sec de ladite résine.

**21.** Utilisation selon l'une des revendications 15 à 20, **caractérisée en ce que** ladite composition est formulée pour une application topique sur la peau ou les phanères.

**Patentansprüche**

**1.** Kosmetische Zusammensetzung, **dadurch gekennzeichnet, dass** sie einen Aucoumeaharz-Extrakt in Anwesenheit eines kosmetisch annehmbaren Trägers enthält.

**2.** Zusammensetzung nach Anspruch 1, **dadurch gekennzeichnet, dass** der Extrakt durch Mazeration des Harzes in einem Lösungsmittel oder einer Mischung von Lösungsmitteln, deren Polaritätsparameter unter oder bei 6 liegt, und anschließende Filtration und nötigenfalls Abdampfung des Lösungsmittels erhalten wird.

**3.** Zusammensetzung nach Anspruch 2, **dadurch gekennzeichnet, dass** das organische Lösungsmittel ausgewählt ist aus der Gruppe bestehend aus aliphatischen $C_6$-$C_{12}$-Kohlenwasserstoffen wie Hexan und Heptan, chlorhaltigen Lösungsmitteln, insbesondere Dichlormethan, Ethern wie Ethylether oder Diisopropylether, Aceton, Estern wie Ethylacetat und Butylacetat, $C_1$-$C_4$-Alkoholen wie Methanol, Ethanol und Isopropanol.

4.  Zusammensetzung nach Anspruch 1, **dadurch gekennzeichnet, dass** der Extrakt durch überkritische Extraktion mit Kohlendioxid erhalten wird.

5.  Zusammensetzung nach einem der vorhergehenden Ansprüche, **dadurch gekennzeichnet, dass** sie 0,01 Gew. % bis 10 Gew.%, insbesondere 0,2 Gew.% bis 1 Gew.% Trockenextrakt des Harzes enthält.

6.  Zusammensetzung nach einem der Ansprüche 1 bis 5, **dadurch gekennzeichnet, dass** sie eine ausreichende Menge des Extrakts enthält, um der Zusammensetzung filmbildende Eigenschaften zu verleihen oder diese zu verbessern.

7.  Zusammensetzung nach einem der Ansprüche 1 bis 6, **dadurch gekennzeichnet, dass** sie in Form einer Krem, insbesondere für Gesicht oder Hände, eines Gels, insbesondere eines Haargels, eines Balsams, insbesondere eines Balsams gegen struppiges Haar, einer Wimperntusche, eines Make-up oder eines Nagelpräparats wie einer Grundierung oder eines Nagellacks mit behandelnder Wirkung vorliegt.

8.  Verwendung eines Aucoumeaharz-Extrakts als kosmetisches Mittel, wobei das Mittel in eine kosmetische Zusammensetzung nach einem der Ansprüche 1 bis 7 eingearbeitet wird.

9.  Verwendung nach Anspruch 8, **dadurch gekennzeichnet, dass** das kosmetische Mittel in ausreichender Menge in die Zusammensetzung eingearbeitet wird, um der Zusammensetzung filmbildende Eigenschaften zu verleihen oder diese zu verbessern.

10. Verwendung nach einem der Ansprüche 8 oder 9, **dadurch gekennzeichnet, dass** die Zusammensetzung eine Pflegezusammensetzung oder eine Schminkzusammensetzung ist.

11. Verwendung nach einem der Ansprüche 8 bis 10, **dadurch gekennzeichnet, dass** das in der Zusammensetzung enthaltene kosmetische Mittel eine hemmende Wirkung gegen Keratonose und/oder Elastase und/oder Phospholipase A2 und/oder Lipoxygenase aufweist.

12. der Keratinstruktur derselben in gutem Zustand, zum Erleichtern des Frisierens und zum Verbessern des Wimpertuschens, bestimmt ist.

13. Verwendung nach einem der Ansprüche 8 bis 12, **dadurch gekennzeichnet, dass** die Zusammensetzung zum Kampf gegen Alterungseffekte auf der Haut bestimmt ist, vor allem durch Konservierung oder Verbesserung der biomechanischen Eigenschaften der Haut, insbesondere ihrer Elastizität, durch Verzögerung der Faltenbildung oder Verringerung der Tiefe derselben und durch Stärkung der Festigkeit der Haut.

14. Verwendung nach einem der Ansprüche 8 bis 13, **dadurch gekennzeichnet, dass** die Zusammensetzung zum Kampf gegen die schädliche Wirkung von freien Radikalen auf die Haut sowie zur Pflege und zum Schminken empfindlicher Haut bestimmt ist, insbesondere durch Milderung oder Behebung von Irritationen, Entzündungen oder Allergien, die sich im Allgemeinen in Hautrötungen, Hautbrennen oder Hautkribbeln äußern.

15. Verwendung eines Aucoumeaharz-Extrakts zur Herstellung einer pharmazeutischen, insbesondere dermatologischen Zusammensetzung zur Verhinderung oder Behandlung von Erkrankungen der Hautanhangsgebilde, die sich in einer Verschlechterung der Keratinstrukturen äußern, zur Behandlung von intrinsischen oder aktinischen Hautalterungseffekten, zur Behandlung der schädlichen Wirkung von freien Radikalen auf die Haut, zur Verhinderung oder Behandlung von allergischen oder entzündlichen Hautsymptomen, wobei der Extrakt in einem pharmazeutisch annehmbaren Träger eingearbeitet wird.

16. Verwendung nach Anspruch 15, **dadurch gekennzeichnet, dass** die Zusammensetzung eine hemmende Wirkung gegen Keratonose und/oder Elastase und/oder Phospholipase A2 und/oder Lipoxygenase aufweist.

17. Verwendung nach einem der Ansprüche 15 oder 16, **dadurch gekennzeichnet, dass** der Extrakt zur Herstellung einer Zusammensetzung verwendet wird, die zur Behandlung oder Verhinderung von Erkrankungen der Hautanhangsgebilde bestimmt ist.

18. Verwendung nach einem der Ansprüche 15 bis 17, **dadurch gekennzeichnet, dass** die pharmazeutische Zusammensetzung weiters mindestens eine aktive Substanz enthält, die für ihre fungizide Aktivität bekannt ist, insbesondere ein Mittel, das gegen Dermatophyten wirkt, die für Nagelpilz verantwortlich sind.

**19.** Verwendung nach einem der Ansprüche 15 bis 18, **dadurch gekennzeichnet, dass** der Extrakt in der Zusammensetzung nach einem Verfahren gemäß einem der Ansprüche 2 bis 4 erhalten ist.

**20.** Verwendung nach einem der Ansprüche 15 bis 19, **dadurch gekennzeichnet, dass** die Zusammensetzung 0,01 Gew.% bis 10 Gew.%, insbesondere 0,2 Gew.% bis 1 Gew.% Trockenextrakt des Harzes enthält.

**21.** Verwendung nach einem der Ansprüche 15 bis 20, **dadurch gekennzeichnet, dass** die Zusammensetzung für topische Anwendung auf der Haut oder auf Hautanhangsgebilden formuliert ist.

**Claims**

**1.** A cosmetic composition, **characterized by** the fact that it contains an extract of okume resin in the presence of a cosmetically acceptable vehicle.

**2.** The composition according to claim 1, **characterized in that** said extract is obtained by maceration of said resin in a solvent or a mixture of solvents having a polarity parameter that is less than or equal to 6, followed by filtering, and where necessary, by evaporation of the solvent.

**3.** The composition according to claim 2, **characterized in that** said organic solvent is selected from the group consisting of $C_6$ to $C_{12}$ aliphatic hydrocarbons such as hexane and heptane, chlorine-containing solvents in particular dichloromethane, ethers such as ethyl ether or diisopropyl ether, acetone, esters such as ethyl acetate and butyl acetate, and $C_1$ to $C_4$ alcohols such as methanol, ethanol, and isopropanol.

**4.** The composition according to claim 1, **characterized in that** said extract is obtained by supercritical carbon dioxide extraction.

**5.** The composition according to one of the preceding claims, **characterized in that** it comprises 0.01% to 10% by weight and in particular 0.2% to 1% by weight dry extract of said resin.

**6.** The composition according to one of claims 1 to 5, **characterized in that** it comprises an effective quantity of said extract for conferring film-forming qualities to said composition or for improving them.

**7.** The composition according to one of claims 1 to 6, **characterized in that** it is presented in the form of a cream, in particular for the face or the hands, a gel, in particular a hair gel, a balm, in particular a untangling balm, a mascara, a foundation, or a preparation for the nails such as a treatment base or a treatment nail varnish.

**8.** The use of an okume resin extract as a cosmetic agent, said agent being incorporated in a cosmetic composition as defined in one of claims 1 to 7.

**9.** The use according to claim 8, **characterized in that** said cosmetic agent is incorporated in said composition in sufficient quantity to confer film-forming qualities to said composition or to improve them.

**10.** The use according to claim 8 or 9, **characterized in that** said composition is a care or makeup composition.

**11.** The use according to one of claims 8 to 10, **characterized in that** said cosmetic agent contained in said composition presents inhibiting action on keratinase and/or elastase and/or phospholipase $A_2$ and/or lipoxygenase.

**12.** The use according to one of claims 8 to 11, **characterized in that** said composition is for treatment of the nails, the hair, or the eyelashes, in particular to maintain them in good condition concerning their keratin structure, to facilitate hair dressing, or to improve eyelash makeup.

**13.** The use according to one of claims 8 to 12, **characterized in that** said composition is intended to combat the effects of aging on the skin, in particular by preserving or improving the biomechanical qualities of the skin, in particular its elasticity, by delaying the appearance of wrinkles or by reducing the depth thereof, and by improving skin firmness.

14. The use according to any one of claims 8 to 13, **characterized in that** said composition is intended to combat the harmful effects of free radicals on the skin, and also to treat or to make up sensitive skins, in particular by attenuating or suppressing phenomena of irritation, inflammation, or allergy, which generally give rise on the skin to red patches, or burning or tingling sensations.

15. The use of an okume resin extract for preparing a pharmaceutical composition, in particular a dermatological composition, for the purposes of preventing or treating complaints of the integuments giving rise to deterioration in keratin structures, of treating the effects of intrinsic or actinic aging of the skin, of treating the harmful effects of free radicals on the skin, of preventing or treating cutaneous manifestations of allergies and inflammations, said extract being incorporated in a pharmaceutically acceptable vehicle.

16. The use according to claim 15, **characterized in that** the composition presents inhibiting activity on keratinase and/or elastase and/or phospholipase $A_2$ and/or lipoxygenase.

17. The use according to one of claims 15 or 16, **characterized in that** said extract is used preparing a composition intended for treating or preventing diseases of the integuments.

18. The use according to one of claims 15 to 17, **characterized in that** said pharmaceutical composition also contains at least one active substance known for its antifungal activity, in particular an agent that is active on the dermatophytes that are responsible for onychomycoses.

19. The use according to one of claims 15 to 18, **characterized in that** said extract contained in said composition is obtained using a method as defined in one of claims 2 to 4.

20. The use according to one of claims 15 to 19, **characterized in that** said composition contains 0.01% to 10% by weight, and in particular 0.2% to 1% by weight of dry extract of said resin.

21. The use according to one of claims 15 to 20, **characterized in that** said composition is formulated for topical application on the skin or on the integuments.

FIG.1

FIG.2

FIG.3

FIG.4